# EUROPÄISCHE PATENTSCHRIFT

(11) **EP 0 852 941 B1**
(45) Veröffentlichungstag und Bekanntmachung des Hinweises auf die Patenterteilung: **14.05.2003**
(21) Anmeldenummer: 97810951.0
(22) Anmeldetag: 05.12.1997
(51) Int. Cl.: A61K 7/00, A61K 7/50

(54) **Kosmetische Präparate in Form einer Nanodispersion**
Nanodispersion cosmetic composition
Composiotn cosmétique sous forme d'une nanodispersion

(30) Priorität: 13.12.1996 CH 306596
(43) Veröffentlichungstag der Anmeldung: 15.07.1998
(73) Patentinhaber: VESIFACT AG, 6340 Baar (CH)
(72) Erfinder: Weder, Hans Georg, Prof. Dr., 8803 Rüschlikon (CH); Weder, Marc Antoine, 8803 Rüschlikon (CH)
(74) Vertreter: Hepp, Dieter

(56) Entgegenhaltungen:
- EP-A- 0 728 460
- WO-A-96/37192
- WO-A-97/21428

## Beschreibung

Gegenstand der vorliegenden Erfindung sind kosmetische Präparate auf der Basis von Nanodispersionen und Verfahren zu ihrer Herstellung.

Kosmetische Präparate zur Hautpflege und zum Hautschutz gibt es in vielen Zubereitungsformen, wie Cremen, Salben, Pasten, Gele, Lotionen, Schäume, Sprays etc.. Neben ihrem eher ästhetischen Nutzen weisen solche dermalen kosmetischen Präparate auch einige Vorteile auf, die sich therapeutisch begründen lassen. So bieten sich diese Präparate wegen ihrer Fixierung an die Epidermis und ihrem Penetrationsvermögen vor allem dann an, wenn sie den Fettund Feuchtigkeitsverlust der Haut infolge von Waschen, Witterungs- und Umwelteinflüssen usw. ausgleichen und die Epidermis gegen Austrocknung schützen sollen. Ein besonders wichtiges Aufgabengebiet haben kosmetische Präparate als sogenannte Lichtschutzpräparate gegen die Einwirkung von UV-Strahlen im Sonnenlicht.

Für kosmetische Präparate eignen sich nur solche Wirkstoffe und Zusatzstoffe, die in der Formulierungsgrundlage löslich oder zumindest ausreichend solubilisiert sind. Ausserdem müssen sie mit den anderen Hilfsstoffen in der Formulierungsgrundlage kompatibel sein. So muss das System aus Wirkstoff und Formulierungsgrundlage ausreichend stabil sein und darf z.B. nicht zu chemischen Reaktionen der Komponenten untereinander und zu Entmischungseffekten neigen. An die Formulierungsgrundlage sind ausserdem noch folgende Anforderungen zu stellen:
- ausreichende homogene Verteilung des Wirkstoffs auf der Hautoberfläche
- ausreichende Feuchtigkeit in den oberen Hautschichten
- Verbesserung der Durchblutung der oberen Hautschichten
- Penetration des Wirkstoffs in die oberen Hautschichten
- Verbesserung der Elastizität der Haut
- Freisetzung des Wirkstoffs aus der Formulierungsgrundlage
- geringe Oxidationsempfindlichkeit

Die in kosmetischen Präparaten verwendete Grundlage hat ausserdem noch folgende subjektive Kriterien zu erfüllen: Angenehme Wahrnehmung nach dem Auftragen des Präparates auf die Haut, gute Netzwirkung, angenehmer Geruch, diskrete oder modische Farbwirkung usw.. Die zahlreichen kommerziell erhältlichen kosmetischen Präparate können die genannten Anforderungen höchstens nur zum Teil erfüllen. Insbesondere die Hautpenetration der verwendeten Wirkstoffe und Lipide ist häufig zu gering. Sehr viele Präparate sind ausserdem wenig wirksam oder gänzlich unwirksam.

Der vorliegenden Erfindung liegt daher im weiteren Sinne die Aufgabe zugrunde, verbesserte kosmetische Präparate bereitzustellen.

Zur Lösung dieser allgemeinen Aufgabenstellung werden gemäss Stand der Technik die aus der pharmazeutischen Technologie bekannten Trägersysteme auf der Basis von Liposomen vorgeschlagen. Diese sollen insbesondere die Penetration von Lipiden in die Epidermis verbessern. Solche Liposomendispersionen mit verschiedenen Einschlussverbindungen und Phospholipiden als Doppelschichtmembranbildner oder sogenannte leere Liposomen ohne Einschlussverbindungen sind in zahlreichen Publikationen beschrieben und bereits klinisch erprobt worden. In der Literatur sind zahlreiche Verfahren zur Herstellung von Liposomendispersionen beschrieben, z.B. durch Behandlung einer wässrigen Phospholipiddispersion mit Ultraschall, Dispersion von Phospholipiden mit Tensiden in wässriger Phase, Auflösen von Phospholipiden in organischen Lösungsmitteln, Entfernen des Lösungsmittels durch Lyophilisierung und Dispersion des Rückstandes in wässriger Phase, Infusionsmethoden oder Umkehrphasenverdampfung. Viele Herstellungsverfahren sind nachteilig, da nur ein Bruchteil der verwendeten Phospholipidmenge Liposomen bildet und diese Liposomen ebenfalls nur einen Bruchteil an Einschlussverbindungen enthalten. Für kosmetische Zwecke stellt man daher fast ausschliesslich "leere" Liposomen ohne Einschlussverbindungen her. Bei den bekannten Herstellungsverfahren können sich zusätzlich auch noch Mischmizellen, Gelstrukturen und Doppelschichtaggregate undefinierbarer Grösse bilden. Bekannt sind ferner Stabilitätsprobleme, stark variierende Grössenverteilung der Liposomen, mangelnde Reproduzierbarkeit der Verfahren selbst, hohe Restmengen an organischen Lösungsmitteln etc..

Zur Lösung der Aufgabenstellung sind daher andere feindisperse Systeme auf der Basis von Lipidgemischen heranzuziehen.

Aus dem Dokument WO 96/37192 sind pharmazeutische oder kosmetische Zusammensetzungen bekannt, die in Kombination mit einem Sphingolipid oder Glycolipid (Ceramid) folgendes enthalten: einen Partialfettsäureester des Polyoxyethylensorbitans, ein Phospholipid, ein Triglycerid und einen therapeutischen Wirkstoff, in Wasser (und eventuell Alkanol) als Trägerflüssigkeit.

Ueberraschend ist nun gefunden worden, dass sich mit einem ähnlichen Hilfsstoffgemisch, vom Typ Partialfettsäureester des Polyoxyethylensorbitans, Phospholipid und Ethanol, auch in Wasser unlösliche, flüssige bis hochviskose, ölige oder jedenfalls in Oel lösliche Wirkstoffe, z.B. fettlösliche Vitamine, therapeutische Oele oder Lichtschutzsubstanzen, in Form einer Dispersion von Nanopartikeln (Nanodispersion) solubilisieren lassen. Der Erfindung liegt im engeren Sinne die Aufgabenstellung zugrunde, für oellösliche kosmetische Wirkstoffe mit geringer Wasserlöslichkeit geeignete wässrige Darreichungsformen herzustellen.

Es wurde überraschenderweise gefunden, dass sich ein besonders homogenes feindisperses System auf der Basis von Nanopartikeln (Nanodispersion) herstellen lässt, indem man einen für die Hautkosmetik geeigneten oellöslichen Wirkstoff im oben beschriebenen Hilfsstoffgemisch löst und die erhaltene Lösung in Wasser dispergiert.

Gegenstand der vorliegenden Erfindung ist ein kosmetisches Präparat in Form einer Nanodispersion nach Anspruch 1.

Eine kosmetische Zusammensetzung mit den Komponenten a), b), c), d) und e) zeichnet sich durch günstige Phaseneigenschaften des solubilisierten Inhaltsstoffs aus. So ist bei vorhandener Opaleszenz und Transparenz im Gegenlicht nur an einer äusserst geringen milchigen Trübung zu erkennen, dass die Dispersion noch physikalische Unterschiede gegenüber dem Idealzustand einer echten molekularen Lösung aufweist. Elektronenmikroskopische Abbildungen zeigen, dass eine Population von mehr als 98 % des solubilisierten Inhaltsstoffs in einer Gaussschen Verteilung als Suspension von Partikeln (Nanopartikel) mit einer Teilchengrösse kleiner als ca. 60 nm (Nanodispersion) vorliegt. Diese Unterschiede gegenüber einer echten Lösung sind aber aufgrund der besonders guten Homogenitätseigenschaften der Dispersion hinnehmbar, die beispielsweise an einer überraschend hohen Lagerstabilität, z. B. keine Entmischung nach mehrmonatiger Lagerung bei Temperaturen bis Raumtemperatur (durch Extrapolation zu erwartende Stabilität länger als zwei Jahre), nachweisbar sind.

Die kosmetische Zusammensetzung eignet sich auch sehr gut für die Verwendung in einem Dosierspray als saubere, praktische Applikationsform. In dem Dosierspray, vorzugsweise einem Pumpdosierspray ohne Treibgas, bleibt die Zusammensetzung steril.

Eine besonders bevorzugte Ausführungsform der Erfindung betrifft
a) mindestens einen für die Hautkosmetik geeigneten Wirkstoff aus der Gruppe der dermal applizierbaren oellöslichen Vitamine, kosmetischen oder therapeutischen Oele und/oder oellöslichen Lichtschutzmittel vom Typ UV-A- und UV-B-Absorber;
b) Polyoxyethylen-(20)-sorbitanmonooleat;
c) gereinigtes Lecithin aus Sojabohnen;
d) Ethanol in der für dermale Applikationen erforderlichen Reinheit;
e) Wasser als Trägerflüssigkeit in der für die dermale Applikation erforderlichen Reinheit; und gegebenenfalls
f) weitere für kosmetische Präparate geeignete Zusatzstoffe.

Die weiter vorn und im folgenden verwendeten Begriffe und Bezeichnungen sind im Rahmen der Beschreibung der vorliegenden Erfindung folgendermassen definiert:

Der Begriff kosmetisches Präparat umfasst dermal oder topisch applizierbare Zubereitungen wie Cremes, z.B. Fett- oder Trockencremes, o/w-, w/o-, w/o/w-Emulsionen, Lotionen, Gele, Sprays, Salben, Pasten und Schäume.

Der Begriff Nanodispersion bezeichnet ein feindisperses System einer suspendierten lipophilen Phase in wässriger oder wässrig alkoholischer Phase. Nanodispersionen sind z.B. anhand elektronenmikroskopischer Abbildungen erkennbar. So sind Populationen von suspendierten Partikeln (Nanopartikel) mit einer Teilchengrösse kleiner als ca. 80 nm (Nanodispersion), vorzugsweise kleiner als 50 nm, vorhanden.

Komponente a) Ein für die Hautkosmetik geeigneter Wirkstoff, eine Wirkstoffzusammensetzung oder ein Wirkstoffextrakt ist ein Inhaltsstoff oder ein Gemisch von Inhaltsstoffen, welches aus der Gruppe der dermal applizierbaren oellöslichen Vitamine oder Placentaextrakte auf Vitaminbasis, kosmetische Oele, sowie oellöslichen Lichtschutzmittel ausgewählt und für die dermale oder topische Verabreichung zugelassen ist.

Dermal applizierbare ölige oder oellösliche Vitamine sind z.B. Vitamin A (Retinol in Form der freien Säure oder ihrer Derivate), Panthenol, Pantothensäure, Folsäure, und Kombinationen davon), Vitamin E (Tocopherol), F: Essentielle Fettsäuren.

Dermal applizierbare kosmetische Oele sind Neutralöl vom Typ Miglyol 812, Aprikosenkernöl, Avocadoöl, Babassuöl, Baumwollsamenöl, Borretschöl, Distelöl, Erdnussöl, Gamma-Oryzanol, Hagebuttenkernöl, Hanföl, Haselnussöl, Johannisbeersamenöl, Jojobaöl, Kirschkernöl, Lachsöl, Leinöl, Maiskeimöl, Makadamianussöl, Mandelöl, Nachtkerzenöl, Nerzöl, Olivenöl, Pekannussöl, Pfirsichkernöl, Pistazienkernöl, Rapsöl, Reiskeimöl, Rizinusöl, Safloröl, Sesamöl, Sojaöl, Sonnenblumennöl, Traubenkernöl, Weizenkeimöl.

Geeignete Lichtschutzmittel sind Filtersubstanzen ("sunscreens"), die UV-Strahlung aus dem Sonnenlicht absorbieren und in Wärme umwandeln können. Je nach der gewünschten Wirkung sind folgende Lichtschutzmittel bevorzugt: Lichtschutzmittel, die selektiv Sonnenbrand erzeugende energiereiche UV-Strahlung im Bereich von ca. 280-315 nm absorbieren (UV-B-Absorber) und den längerwelligen Bereich von ca. 315-400 nm (UV-A-Bereich) transmittieren, sowie Lichtschutzmittel, welche nur die längerwellige Strahlung des UV-A-Bereichs von 315-400 nm absorbieren (UV-A-Absorber).

Geeignete Lichtschutzmittel sind vorzugsweise aus der Gruppe der Anthranilsäurederivate, Salicylsäurederivate, Zimtsäureesterderivate, Cumarinderivate, o-Aminobenzoesäurederivate, Benzophenon und Benzylidencampher ausgewählt.

Spezifische UV-B-Absorber sind z.B. 4-Aminobenzoesäure, 2-Ethylhexyl-4-methoxy-cinnamat, 2-Ethylhexylsalicylat, oder UV-A-Absorber, z.B. Benzophenon-4, -3, oder -10. Geeignete Lichtschutzmittel sind in *Pflegekosmetik: Ein Leitfaden, W.Raab, U.Kindl; Govi-Verlage, D-Frankfurt 1991* und in *Ullmann's Encyclopedia of Industrial Chemistry, VCH Publishers, Fifth Complete Revised Edition, Volume A 24, Entry Skin Cosmetics beschrieben.*

Die Komponente a) - ist in der weiter vorn definierten kosmetischen Zusammensetzung in einer bevorzugten Dosierung von 0,1 bis 20 Gewichtsprozent, bezogen auf das Gesamtgewicht der Formulierung, vorhanden.

Die Komponente b) - Partialfettsäureester des Polyoxyethylensorbitans - besteht vorzugsweise aus einem im wesentlichen reinen oder dem Gemisch verschiedener Ester des Sorbitans, worin die Struktur der Fettsäuregruppen und die Länge der Polyoxyethylenketten variieren. Das Sorbitan ist vorzugsweise durch drei hydrophile Polyoxyethylenketten veräthert und durch eine hydrophobe Fettsäuregruppe verestert. Das Sorbitan kann aber auch nur durch ein oder zwei hydrophile Polyoxyethylenketten veräthert und entsprechend durch drei oder zwei hydrophobe Fettsäuregruppen verestert sein. Insgesamt ist der Sorbitangrundkörper durch mindestens ein bis maximal drei hydrophile Gruppen und entsprechend durch maximal drei und mindestens eine hydrophobe Gruppe substituiert, wobei der Begriff hydrophile Gruppe die Polyoxyethylenketten und der Begriff hydrophobe Gruppe Fettsäuregruppen umfasst.

Die Polyoxyethylenkette ist geradkettig und weist vorzugsweise 4-10, insbesondere 4-8, Ethylenoxideinheiten auf. Die Estergruppen am Sorbitangrundkörper sind von einer gesättigten oder ungesättigten, geradkettigen Carbonsäure und gerader Anzahl von 8-20 C-Atomen abgeleitet. Die von dieser Carbonsäure abgeleitete Estergruppe ist vorzugsweise geradkettig mit 12, 14, 16 und 18 C-Atomen, z.B. n-Dodecanoyl, n-Tetradecanoyl, n-Hexadecanoyl oder n-Octadecanoyl. Die von einer ungesättigten Carbonsäure mit gerader Anzahl von 8-20 C-Atomen abgeleitete Estergruppe ist vorzugsweise geradkettig mit 12, 14, 16 und 18 C-Atomen, z.B. Oleoyl. Die genannten Ester des Sorbitans erfüllen die in der Britischen Pharmakopöe (spezielle Monographie) oder Ph.Helv.VII genannten Angaben. Es gelten insbesondere die von den genannten Herstellern publizierten Produktbeschreibungen mit den Angaben auf Datenblättern für das betreffende Produkt, insbesondere Spezifikationen wie Form, Farbe, HLB-Wert, Viskosität, Steigschmelzpunkt und Löslichkeit.

Geeignete Partialfettsäureester des Polyoxyethylensorbitans sind unter dem Wortzeichen Tween® der Fa.ICI kommerziell erhältlich und den chemischen Bezeichnungen Polyoxyethylen-(20 oder 4)-sorbitanmonolaurat (TWEEN 20 und 21), Polyoxyethylen-(20)-sorbitanmonopalmitat oder -monostearat (TWEEN 40 und 60), Polyoxyethylen-(4 oder 20)-sorbitanmonostearat oder -tristearat (TWEEN 61 und 65), Polyoxyethylen-(20 oder 5)-sorbitanmonooleat (TWEEN 80 oder 81) oder Polyoxyethylen-(20)-sorbitantrioleat (TWEEN 85) bekannt.

In einer besonders bevorzugten Ausführungsform der Erfindung verwendet man als Komponente b) Polyoxyethylen-(20)-sorbitanmonooleat (TWEEN 80).

Die Komponente b) ist in der kosmetischen Zusammensetzung in einem Mengenanteil (bezogen auf das Gesamtgewicht der Formulierung) von ca. 0,1 % bis ca. 5 %, vorzugsweise 0,5 % bis 3 %, vorhanden.

Komponente c) - Phospholipid der Formel I. Die Nomenklatur der Phospholipide (I) und die Bezifferung der C-Atome erfolgt anhand der in Eur. J. of Biochem. 79, 11-21 (1977) "Nomenclature of Lipids" von der IUPAC-IUB Commission on Biochemical Nomenclature (CBN) gegebenen Empfehlungen (sn-Nomenklatur, stereospecific numbering).

R₁ und R₂ mit den Bedeutungen C₁₀₋₂₀-Acyl sind vorzugsweise geradkettiges C₁₀₋₂₀-Alkanoyl mit einer geraden Anzahl an C-Atomen und geradkettiges C₁₀₋₂₀-Alkenoyl mit einer Doppelbindung und einer geraden Anzahl an C-Atomen.

Geradkettiges C₁₀₋₂₀-Alkanoyl R₁ und R₂ mit einer geraden Anzahl an C-Atomen sind beispielsweise n-Dodecanoyl, n-Tetradecanoyl, n-Hexadecanoyl oder n-Octadecanoyl.

Geradkettiges C₁₀₋₂₀-Alkenoyl R₁ und R₂ mit einer Doppelbindung und einer geraden Anzahl an C-Atomen sind beispielsweise 6-cis- oder 6-trans-, 9-cis- oder 9-trans-Dodecenoyl, -Tetradecenoyl, -Hexadecenoyl, -Octadecenoyl oder -Icosenoyl, insbesondere 9-cis-Octa-decenoyl (Oleoyl), ferner 9,12-cis-Octadecadienoyl oder 9,12,15-cis-Octadecatrienoyl.

Ein Phospholipid (I), worin R₃ 2-Trimethylamino-1-ethyl bedeutet, wird mit dem Trivialnamen Lecithin und ein Phospholipid (I), worin R₃ 2-Amino-1-ethyl bedeutet, mit dem Trivialnamen Kephalin bezeichnet. Geeignet sind beispielsweise natürlich vorkommendes Kephalin oder Lecithin, z.B. Kephalin oder Lecithin aus Sojabohnen oder Hühnerei mit verschiedenen oder identischen Acylgruppen R₁ und R₂, oder Mischungen davon.

Das Phospholipid (I) kann aber auch synthetischen Ursprungs sein. Unter dem Begriff synthetisches Phospholipid definiert man Phospholipide, welche bezüglich R₁ und R₂ eine einheitliche Zusammensetzung haben. Solche synthetischen Phospholipide sind vorzugsweise die weiter vorn definierten Lecithine und Kephaline, deren Acylgruppen R₁ und R₂ eine definierte Struktur haben und von einer definierten Fettsäure mit einem Reinheitsgrad höher als ca. 95% abgeleitet sind. R₁ und R₂ können gleich oder verschieden und ungesättigt oder gesättigt sein. Bevorzugt ist R₁ gesättigt, z.B. n-Hexadecanoyl, und R₂ ungesättigt, z.B. 9-cis-Octadecenoyl (Oleoyl).

Der Begriff "natürlich vorkommendes" Phospholipid (I) definiert Phospholipide, welche bezüglich R₁ und R₂ keine einheitliche Zusammensetzung haben. Solche natürlichen Phospholipide sind ebenfalls Lecithine und Kephaline, deren Acylgruppen R₁ und R₂ strukturell undefinierbar und von natürlich vorkommenden Fettsäuregemischen abgeleitet sind.

Die Forderung "im wesentlichen reines" Phospholipid (I) definiert einen Reinheitsgrad von mehr als 90%(Gew.), vorzugsweise mehr als 95%, des Phospholipids (I), welcher anhand geeigneter Bestimmungsmethoden, z.B. papierchromatographisch, mit Dünnschichtchromatographie, mit HPLC oder enzymatischem Farbtest, nachweisbar ist.

In einem Phospholipid (I) ist R₃ mit der Bedeutung C₁₋₄-Alkyl beispielsweise Methyl oder Ethyl. Die Bedeutung Methyl ist bevorzugt.

R₃ mit den Bedeutungen C₁₋₅-Alkyl substituiert durch Carboxy, C₂₋₅-Alkyl substituiert durch Hydroxy oder C₂₋₅-Alkyl substituiert durch Carboxy oder Hydroxy sind beispielsweise 2-Hydroxyethyl, 2,3-Dihydroxy-n-propyl, Carboxymethyl, 1- oder 2-Carboxyethyl, Dicarboxymethyl, 2-Carboxy-2-hydroxyethyl oder 3-Carboxy-2,3-dihydroxy-n-propyl.

R₃ mit der Bedeutung C₂₋₅-Alkyl substituiert durch Carboxy und Amino ist z.B. 3-Amino-3-carboxy-n-propyl oder 2-Amino-2-carboxy-n-propyl, vorzugsweise 2-Amino-2-carboxyethyl. Phospholipide (I) mit diesen Gruppen können in Salzform, z.B. als Natrium- oder Kaliumsalz, vorliegen.

Phospholipide (I), worin R₃ die Inositol- oder die Glycerylgruppe bedeutet, sind unter den Bezeichnungen Phosphatidylinositol und Phosphatidylglycerol bekannt.

Für die Acylreste in den Phospholipiden (I) sind auch die in Klammern angegebenen Bezeichnungen gebräuchlich:

9-cis-Dodecenoyl (Lauroleoyl), 9-cis-Tetradecenoyl (Myristoleoyl), 9-cis-Hexadecenoyl (Palmitoleoyl), 6-cis-Octadecenoyl (Petroseloyl), 6-trans-Octadecenoyl (Petroselaidoyl), 9-cis-Octadecenoyl (Oleoyl), 9-trans-Octadecenoyl (Elaidoyl), 9,12-cis-Octadecadienoyl (Linoleoyl), 9,12,15-cis-Octadecatrienoyl (Linolenoyl), 11-cis-Octadecenoyl (Vaccenoyl), 9-cis-Icosenoyl (Gadoleoyl), 5,8,11-,14-cis-Eicosatetraenoyl (Arachidonoyl), n-Dodecanoyl (Lauroyl), n-Tetradecanoyl (Myristoyl), n-Hexadecanoyl (Palmitoyl), n-Octadecanoyl (Stearoyl), n-Icosanoyl (Arachidoyl), n-Docosanoyl (Behenoyl), n-Tetracosanoyl (Lignoceroyl).

Ein Salz des Phospholipids (I) ist vorzugsweise pharmazeutisch annehmbar. Salze definieren sich durch die Existenz von salzbildenden Gruppen im Substituenten R₃ sowie durch die freie Hydroxygruppe am Phosphor. Möglich ist ebenfalls die Bildung von inneren Salzen. Bevorzugt sind Alkalimetallsalze, insbesondere Natriumsalze.

Die Komponente c) wird in einer bevorzugten Konzentration von ca. 0,1 bis 5 Gew.-%, vorzugsweise 0,5 bis 3 Gew.-%, bezogen auf das Gesamtgewicht der Formulierung, hinzugesetzt. In einer besonders bevorzugten Ausführungsform verwendet man gereinigtes Lecithin aus Sojabohnen der Qualität LIPOID S 100.

Die Komponente d) besteht vorzugsweise aus Ethanolum absolutum.

Die Komponente e) - Wasser in der für die dermale Applikation erforderlichen Reinheit - ist nach den geltenden Vorschriften der nationalen Pharmakopöen keim- und, falls nötig, pyrogenfrei.

Die Komponente f) - für dermale Darreichungsformen geeignete Hilfsstoffe - ist als fakultative Komponente enthalten. Solche Hilfsstoffe sind in o/w-, w/o-, w/o/w-Emulsionen, Cremen, Salben, Gelen, Lotionen, Pasten, Sprühschäumen, Sprays, Aerosolen, Tinkturen oder Lotionen enthalten.

Geeignet sind insbesondere Emulgatoren, Fettsäuresalze, z.B. Natriumstearat, emulgierende Gemische aus Fettsäuren und deren Salzen, z.B. das Gemisch aus Stearinsäure und Natriumstearat, ethoxylierte Fettalkohole, z.B. Polyoxyethylen(20)-Cetyl-Stearylether, hydriertes ethoxyliertes Castoröl, z.B. Polyoxyethylen(7)-hydriertes Castoröl, fettähnliche Emulgatoren, z.B. Wollwachs, Wollwachsalkohole, höhere Fettalkohole oder Glycerinpartialester oder Sorbitanpartialester vom Typ Glycerinmonostearat oder Sorbitanmonooleat, Orthophosphorsäureester, z.B. Cetylphosphat, oder Tenside auf Siliconbasis, z.B. Dimethiconpolyol. Lipophile Bestandteile, Siliconöle oder Siliconwachse, Fettsäureester vom Typ Cetylpalmitat, Isostearylisostearat, Isopropylpalmitat oder -myristat, Mineralöl, Vaseline, natürliche Wachse oder Guerbetalkohole, ferner Konservierungsmittel, z.B. Benzalkoniumchlorid, Phenoxyethanol, ferner Benzoesäure oder deren Salze, 4-Hydroxybenzoesäureester (PHB-Ester), Phenole, z.B. tert.-Butyl-4-methoxy- oder Di-tert.-butyl-4-methylphenol, Benzylalkohol, 4-Chlor- oder 2,4-Dichlorbenzylalkohol, 2-Phenylethanol, Chlorhexidindiacetat oder -digluconat, Thiabendazol, Cetyltriammoniumbromid, Cetyl-pyridiniumbromid, Phenododeciniumbromid oder Sorbinsäure, Stabilisatoren, welche eine Entmischung (Demulgierung) verhindern, z.B. Tragant, Stärke, Dextrine, Pektine, Alginate, Celluloseester, Eiweisse oder Polyvinyalkohol, Regulatoren der Hautfeuchtigkeit, z.B. Aminosäuren, wie, Alanin, Arginin, Glutaminsäure, Glycin, Histidin, Serin oder 2-Pyrrolidon-5-carbonsäure oder Natriumlactat, Collagen-Hydrolysate, Zucker (Pentosen) oder Reaktionsprodukte von D-Glucose und L-Glutaminsäure, Antioxidantien, z.B. Ascorbinsäure, Cystein, Sulfite, z.B. Natriumbisulfit, Thioglycol oder Glutathion, ätherische Öle zur Verbesserung des Geruchs, z.B. Menthol, Orangen-, Pomeranzen-, Mandarinen- oder Citronenöl, Fliessmittel , ferner Pigmente zur Verbesserung des farblichen Aussehens, z.B. Talk, Zinkoxid, Kaolin, Titandioxid, Eisenoxid, Chromoxide, Manganviolett, Ultramarin oder Farbstoffe mit Glimmereffekt, z.B. Mica.

Der Hilfsstoffgehalt der Komponente f) kann in weiten Bereichen variieren und hängt von der Art und dem ästhetischen Charakter der gewünschten Zubereitungsform ab. Als allgemeine Obergrenze wird ein Höchstgehalt von ca. 20 Gew.-% angesehen.

Zur Verbesserung der Hautpenetration der Inhaltsstoffe kann man sogenannte Penetriermittel (flux enhancer) hinzufügen, welche die Penetration in die oberen Hautschichten verbessern. Geeignete Penetriermittel sind z.B. Amide, z.B. N,N-Dimethyllauroylamid, 1-n-Dodecylazacycloheptan-2-on (Azone®, Nelson), N-Methyl-2-pyrrolidon, Harnstoff oder Isopropylmyristat.

Ebenfalls Gegenstand der Erfindung ist das Verfahren zur Herstellung der kosmetischen Zusammensetzung gemäss Anspruch 1, welches dadurch gekennzeichnet ist, dass man die Komponente c) - Phospholipid - in der Komponente d) - Ethanol - löst und anschliessend die Komponente b) - Partialfettsäureester des Polyoxyethylensorbitans - sowie die Komponente a) - Wirkstoff - hinzufügt und das Gemisch bei Raumtemperatur oder erhöhter Temperatur rührt.

Gemäss diesem Verfahren stellt man eine besonders homogene, dermal applizierbare Dispersion mit Nanopartikeln der lipophilen Inhaltsstoffe a) her.

Man erhält die Nanodispersion durch Hinzufügen der lipophilen Phase ("Ölphase"), bestehend aus den Inhaltsstoffen der Komponente a), sowie den Komponenten b), c), d) und gegebenenfalls lipophilen Hilfsstoffen - Komponente f) - zur wässrigen Phase - Komponente e), die gegebenenfalls weitere hydrophile Hilfsstoffe - Komponente f) - enthält. Man mischt das Gemisch zwei bis drei Stunden lang mit einem Magnetrührer oder einem statischen Mischer. Der Mischvorgang erfolgt vorzugsweise bei Raumtemperatur oder leichtem Erwärmen bei Temperaturen bis zu 45°C.

Die so erhältliche Dispersion lässt sich als Dispersion von kolloiden Nanopartikeln oder vereinfacht als Nanodispersion definieren. Aufgrund von Laser-Lichtstreumessungen und Aufnahmen im Elektronenmikroskop sind die in der Dispersion vorhandenen kolloiden Partikel von anderen Gebilden wie Flüssigkristallen, Mizellen, Umkehrmizellen oder Liposomen unterscheidbar. Für die statistische Mehrzahl von mehr als 95%, vorzugsweise mehr als 99%, ist eine mittlere Partikelgrösse kleiner als 80 nm charakteristisch.

Zur Charakterisierung der erhältlichen Nanodispersion sind an sich bekannte Methoden geeignet, z.B. optische Beurteilung: schwache bis starke Opaleszenz des Präparats ist leicht erkennbar (Hinweis auf durchschnittliche Partikelgrösse kleiner als 50 nm), Laser-Lichtstreuung (Bestimmung der Partikelgrösse und Homogenität); Elektronenmikroskopie (Gefrierbruch- und Negativkontrasttechnik).

Die folgenden Beispiele illustrieren die Erfindung; Prozentangaben in Gewichtsprozenten; die relevanten physikalisch-chemischen Parameter, wie Nanopartikelgrösse und Nanopartikelverteilung (Laser-Lichtstreumessungen im nm-Bereich), (Partikelzählgerät nach USP im µm-Bereich), Viskosität und Wirkstoffgehalt der Formulierungen sind im Anschluss an die Beispiele in einer Tabelle zusammengefasst

Beispiel 1: Rezeptur für eine Vitamin E-Nanodispersion.

| | |
|---|---|
| 94.701 % | Aqua purificata |
| 2.000 % | Tocopheroli acetas (alpha) Ph.Eur.III |
| 1.350 % | Polysorbatum 80 Ph.Eur.III |
| 1.000 % | Lipoid S 100 |
| 0.650 % | Ethanolum absolutum |
| 0.272 % | Natrii dihydrogenophosphas dihydricus Ph.Eur.III |
| 0.027 % | Dinatrii phosphas dihydricus Ph.Eur.III |

Man löst Lipoid S 100 in Ethanol und fügt Polysorbatum 80 und Tocopheroliacetas hinzu und gibt diese Oelphase zur wässrigen Phase - die beiden Puffersalze enthaltend - hinzu und rührt zwei bis drei Stunden lang (200 - 300 Upm) bei Raumtemperatur, bis die Mischung transparent und schwach opaleszent ist.

Beispiel 2: Rezeptur für eine Vitamin A-Nanodispersion.

| | |
|---|---|
| 96.129 % | Aqua purificata |
| 1.497 % | Polysorbatum 80 Ph.Eur.III |
| 1.000 % | Lipoid S 100 |
| 0.650 % | Ethanolum absolutum |
| 0.325 % | Vitamin A Palmitat 1.7M IU/g (Toc) |
| 0.272 % | Natrii dihydrogenophosphas dihydricus Ph.Eur.III |
| 0.100 % | Tocopheroli acetas (alpha) Ph.Eur.III |
| 0.027 % | Dinatrii phosphas dihydricus Ph.Eur.III |

Das Vorgehen erfolgt in zum Beispiel 1 analoger Weise.

Beispiel 3: Rezeptur für eine Makadamianussöl-Nanodispersion

| | |
|---|---|
| 92.560 % | Aqua purificata |
| 2.133 % | Makadamianussöl raffiniert |
| 1.800 % | Polysorbatum 80 Ph.Eur.III |
| 1.333 % | Lipoid S 100 |
| 0.867 % | Ethanolum absolutum |
| 0.533 % | Tocopheroli acetas (alpha) Ph.Eur.III |
| 0.500 % | Phenoxyethanol |
| 0.247 % | Kalii dihydrogenophosphas DAB10 |
| 0.027 % | Dinatrii phosphas dihydricus Ph.Eur.III |

Das Vorgehen erfolgt in zum Beispiel I analoger Weise.

Beispiel 4: Rezeptur für eine Johannisbeersamenöl-Nanodispersion.

| | |
|---|---|
| 94.226 % | Aqua purificata |
| 2.000 % | Johannisbeersamenöl raffiniert |
| 1.350 % | Polysorbatum 80 Ph.Eur.III |
| 1.000 % | Lipoid S 100 |
| 0.650 % | Ethanolum absolutum |
| 0.500 % | Phenoxyethanol |
| 0.247 % | Kalii dihydrogenophosphas DAB10 |
| 0.027 % | Dinatrii phosphas dihydricus Ph.Eur.III |

Das Vorgehen erfolgt in zum Beispiel 1 analoger Weise.

Beispiel 5: Rezeptur für eine Mandelöl-Nanodispersion

| | |
|---|---|
| 94.226 % | Aqua purificata |
| 2.000 % | Mandelöl raffiniert Ph.Eur.III |
| 1.350 % | Polysorbatum 80 Ph.Eur.III |
| 1.000 % | Lipoid S 100 |
| 0.650 % | Ethanolum absolutum |
| 0.500 % | Phenoxyethanol |
| 0.247 % | Kalii dihydrogenophosphas DAB10 |
| 0.027 % | Dinatrii phosphas dihydricus Ph.Eur.E |

Das Vorgehen erfolgt in zum Beispiel 1 analoger Weise.

Beispiel 6: Rezeptur für eine Parsol MCX-Nanodispersion

| | |
|---|---|
| 95.000 % | Aqua purificata |
| 2.500 % | Parsol MCX |
| 0.944 % | Lipoid S 100 |
| 0.723 % | Ethanolum absolutum |
| 0.585 % | Polysorbatum 80 Ph.Eur.III |
| 0.248 % | Miglyol 812 Neutralöl |

Das Vorgehen erfolgt in zum Beispiel 1 analoger Weise.

Beispiel 7: Rezeptur für eine Dexpanthenol 5%-Nanodispersion Dosiersprayformulierung.

| | |
|---|---|
| 88.315 % | Aqua purificata |
| 6.667 % | D-Panthenol 75L USP23 |
| 1.434 % | Miglyol 812 Neutralöl DAB10 |
| 1.413 % | Polysorbatum 80 Ph.Eur.III |
| 0.717 % | Lipoid S 100 |
| 0.600 % | Phenoxyethanol |
| 0.591 % | Ethanolum absolutum |
| 0.237 % | Kalii dihydrogenophosphas DAB10 |
| 0.026 % | Dinatrii phosphas dihydricus Ph.Eur.III |

Das Vorgehen erfolgt in zum Beispiel 1 analoger Weise, wobei die sterilfiltrierte Nanodispersion in Pumpdosiersprays mit 30 ml Inhalt konfektioniert wird.

Die physikalisch-chemischen Parameter der kosmetischen Formulierungen sind in der nachfolgenden Tabelle zusammengestellt:

| Beispiel | Chargen-Nr. und -Grösse | Laser-Lichtstreuung¹⁾ Nanopartikelgrösse±S.D. | Viskosität |
|---|---|---|---|
| | [kg] | [nm] | [mPä*s] |
| 1 | 503.001 | 30.5 ± 4.4 | 1.4 |
| | 5.0 | | |
| 2 | 305.002 | 21.5 ± 4.4 | 1.2 |
| | 5.0 | | |
| 3 | 307.002 | 24.5 ± 5.7 | 1.3 |
| | 5.0 | | |
| 4 | 310.001 | 19.5 ± 7.0 | 1.3 |
| | 5.0 | | |
| 5 | 312.001 | 35.7 ± 6.0 | 1.3 |
| | 5.0 | | |
| 6 | 990.023 | 71.4 ± 13.8 | 1.2 |
| | 0.5 | | |
| 7 | 905.012 | 21.2 ± 4.2 | 1.6 |
| | 3.6 | | |

| | | | |
|---|---|---|---|
| ¹⁾Nicomp 370 Sumicron Particle Sizer; Nicomp Distribution Analysis Number Weighting | | | |

## Patentansprüche

1. Kosmetisches Präparat in Form einer Nanodispersion, bestehend aus
a) mindestens einen für die Hautkosmetik geeigneten, oellöslichen Wirkstoff ausgewählt aus der Gruppe bestehend aus dermal applizierbaren Vitaminen aus der Gruppe bestehend aus Vitamin A, Panthenol, Pantothensäure, Folsciure, Vitamin E und essentiellen Fettsäuren kosmetischen Oelen und oellöslichen LichtSchutzmitteln,
b) einen Partialfettsäureester des Polyoxyethylensorbitans,
c) mindestens ein im wesentlichen reines Phospholipid der Formel 1 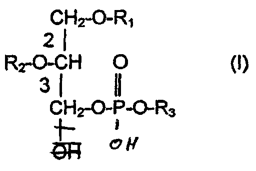 worin R₁ C₁₀₋₂₀-Acyl, R₂ Wasserstoff oder C₁₀₋₂₀-Acyl, R₃ Wasserstoff, 2-Trimethylamino-1-ethyl, 2-Amino-1-ethyl, C₁₋₄-Alkyl, C₁₋₅-Alkyl substituiert durch Carboxy, C₂₋₅-Alkyl substituiert durch Hydroxy, C₂₋₅-Alkyl substituiert durch Carboxy und Hydroxy oder C₂₋₅-Alkyl substituiert durch Carboxy und Amino, die Inositol- oder die Glycerylgruppe bedeuten, oder Salze dieser Verbindungen,
d) Ethanol in der für dermale Applikationen erforderlichen Reinheit,
e) Wasser als Trägerflüssigkeit in der für dermale Applikationen erforderlichen Reinheit, und
f) gegebenenfalls weitere für kosmetische Präparate geeignete Zusatzstoffe ausgewählt aus der Gruppe, bestehend aus Emulgatoren. Siliconölen oder Siliconwachsen, Fettsäureestern vom Typ Cetylpalmitat, Isostearylisostearat, Isopropylpalmitat oder - myristat, Mineralölen, natürlichen Wachsen oder Guerbetalkoholen, Konservierungsmitteln, Stabilisatoren welche eine Entmischung (Demulgierung) verhindern, Regulatoren der Hautfeuchtigkeit, z.B. Aminosäuren, Antioxidantien, ätherischen Ölen, Fliessmittel, Pigmenten, Farbstoffer mit Glimmer effekt und Penetriermitteln (flux enhancer).

2. Kosmetisches Präparat nach Anspruch 1, **dadurch gekennzeichnet, dass** die Komponente (a) in dem Präparat in einer Dosierung von 0.1 bis 20 Gewichtsprozent, bezogen auf das Gesamtgewicht des Präparates, vorhanden ist.

3. Kosmetisches Präparat nach Anspruch 1 oder 2, **dadurch gekennzeichnet, dass** die Komponente (b) in dem Präparat in einer Dosierung von 0,1 bis 5 Gewichtsprozent, bezogen auf das Gesamtgewicht des Präparates, vorhanden ist.

4. Kosmetisches Präparat nach einem der Ansprüche 1 bis 3, **dadurch gekennzeichnet, dass** die Komponente (c) in dem Präparat in einer Dosierung von 0,1 bis 5 Gewichtsprozent, bezogen auf das Gesamtgewicht des Präparates, vorhanden ist.

5. Kosmetisches Präparat nach Anspruch 1, **dadurch gekennzeichnet, dass** der für die Hautkosmetik geeignete Wirkstoff ausgewählt ist aus der Gruppe bestehend aus dermal applizierbaren oellöslichen Vitaminen, kosmetischen oder therapeutischen Oelen und oellöslichen Lichtschutzmitteln vom Typ UV-A- und W-B-Absorber.

6. Kosmetisches Präparat nach einem der Ansprüche 1 bis 5, **dadurch gekennzeichnet, dass** der Partialfettsäureester des Polyoxyethylensorbitans (b) Polyoxyethylen-(20)-sorbitanmonooleat ist.

7. Kosmetisches Präparat nach einem der Ansprüche 1 bis 6, **dadurch gekennzeichnet, dass** das Phospholipid (c) gereinigtes Lecithin aus Sojabohnen ist.

8. Verwendung des kosmetischen Präparates nach einem der Ansprüche 1 bis 7 in einem Dosierspray.

9. Verwendung nach Anspruch 8, **dadurch gekennzeichnet, dass** der Dosierspray ein Pumpspray ist.

10. Verfahren zur Herstellung eines kosmetischen Präparates gemäss einem der Ansprüche 1 bis 9, **dadurch gekennzeichnet, dass** man die Komponente (c) - Phospholipid - in der Komponente (d) - Ethanol - löst und anschliessend die Komponente (b) - Partialfettsäureester des Polyoxyethylensorbitans - sowie die Komponente (a) - Wirkstoff - hinzufügt und die so erhaltene, gegebenenenfalls lipophile Komponenten f) umfassende Oelphase in die wässrige, gegebenenfalls hydrophile Komponenten f) umfassende Phase - Komponente (e) - gibt und rührt

11. Kosmetisches Präparat in Form einer Nanodispersion, erhältlich nach dem Verfahren gemäss Anspruch 10.

## Claims

1. Cosmetic preparation in the form of a nano-dispersion, comprising
a) at least one oil-soluble active ingredient suitable for skin cosmetics, selected from the group comprising dermally applicable vitamins from the group comprising vitamin A, panthenol, pantothenic acid, folic acid, vitamin E and essential fatty acids, and oil-soluble sun screens,
b) a partial fatty acid ester of polyoxyethylene sorbitan,
c) at least one essentially pure phospholipid of the formula 1 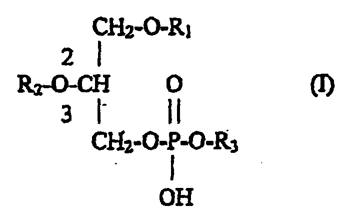 where R₁ means C₁₀₋₂₀-acyl, R₂ means hydrogen or C₁₀₋₂₀-acyl, R₃ means hydrogen, 2-trimethylamino-1-ethyl, 2-amino-1-ethyl, C₁₋₄-alkyl, C₁₋₅-alkyl substituted by carboxy, C₂₋₅-alkyl substituted by hydroxy, C₂₋₅-alkyl substituted by carboxy and hydroxy, or C₂₋₅-alkyl substituted by carboxy and amino, which mean inositol or the glyceryl group, or salts of these compounds,
d) ethanol in the purity required for dermal applications,
e) water as carrier fluid in the purity required for dermal applications, and
f) if necessary further additives suitable for cosmetic preparations, selected from the group comprising emulsifiers, silicon oils or silicon "waxes, fatty acid esters of the type cetyl palmitate, isostearylisostearate, isopropylpalmitate or -myristate, mineral oils, natural waxes or guerbet alcohols, preservatives, stabilisers that prevent separation (demulsification), regulators of skin moisture, e.g. amino acids, antioxidants, essential oils, flow agents, pigments, dyes with glitter effect, and penetration agents (flux enhancers).

2. Cosmetic preparation in accordance with claim 1, **characterised in that** the component (a) in the preparation is present in a dosage of 0.1 to 20 per cent by weight in relation to the total weight of the preparation.

3. Cosmetic preparation in accordance with claim 1 or 2, **characterised in that** the component (b) in the preparation is present in a dosage of 0.1 to 5 per cent by weight in relation to the total weight of the preparation.

4. Cosmetic preparation in accordance with one of the claims I to 3, **characterised in that** the component (c) in the preparation is present in a dosage of 0.1 to 5 per cent by weight in relation to the total weight of the preparation.

5. Cosmetic preparation in accordance with claim 1, **characterised in that** the active ingredient suitable for skin cosmetics is selected from the group comprising dermally-applicable oil-soluble vitamins, cosmetic or therapeutic oils and oil-soluble sun screens of the type UV-A and UV-B absorbers.

6. Cosmetic preparation in accordance with one of the claims 1 to 5, **characterised in that** the partial fatty acid ester of the polyoxyethylene sorbitan (b) is polyoxyethylene-(20)-sorbitan monooleate.

7. Cosmetic preparation in accordance with one of the claims 1 to 6, **characterised in that** the phospholipid (c) is purified lecithin from soya beans.

8. Use of the cosmetic preparation according to one of the claims 1 to 7 in a dosing spray.

9. Use in accordance with claim 8, **characterised in that** the dosing spray is a pump spray.

10. Method for producing a cosmetic preparation in accordance with one of the claims 1 to 9, **characterised in that** the component (c) -phospholipid - is dissolved in the component (d) - ethanol - and subsequently the component (b) - partial fatty acid of polyoxyethylene sorbitan - and component (a) - active ingredient - are added, and the oil phase which is thus obtained, possibly containing lipophilic components (f) - is added to the aqueous phase - component (e) - possibly containing lipophilic components (f), and stirred.

11. Cosmetic preparation in the form of a nano-dispersion, obtainable according to the method in accordance with claim 10.

## Revendications

1. Composition cosmétique sous forme d'une nanodispersion, constituée
a) d'au moins un agent actif approprié pour un cosmétique de la peau, soluble dans l'huile, choisi dans le groupe constitué des vitamines pouvant être appliquées par voie dermique et du groupe constitué de la vitamine A, du panthénol, de l'acide pantothénique, de l'acide folique, de la vitamine E et des acides gras essentiels, des huiles cosmétiques et des agents de protection contre la lumière solubles dans l'huile,
b) d'un ester partiel d'acide gras du polyoxyéthylènesorbitane,
c) d'au moins un phospholipide essentiellement pur de formule I 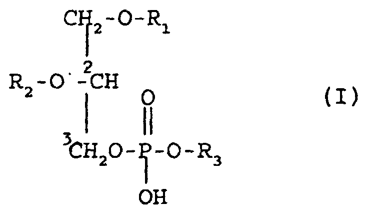 dans laquelle R₁ signifie un groupe acyle en C₁₀ à C₂₀, R₂ signifie un atome d'hydrogène ou un groupe acyle en C₁₀ à C₂₀, R₃ signifie un atome d'hydrogène, un groupe 2-triméthylamino-1-éthyle, 2-amino-1-éthyle, alkyle en C₁ à C₄, alkyle en C₁ à C₅, substitué par un groupe carboxy, alkyle en C₂ à C₅ substitué par un groupe hydroxy, alkyle en C₂ à C₅ substitué par un groupe carboxy et un groupe hydroxy ou alkyle en C₂ à C₅ substitué par un groupe carboxy et un groupe amino, le groupe inositol ou glycéryle, ou des sels de ces composés,
d) de l'éthanol présentant la pureté nécessaire pour une application par voie dermique,
e) de l'eau comme liquide support présentant la pureté nécessaire pour une application par voie dermique,
f) le cas échéant d'autres additifs appropriés pour des compositions cosmétiques, choisis parmi le groupe constitué des émulsifiants, des huiles de silicone ou des cires de silicone, des esters d'acide gras du type palmitate de cétyle, isostéarate d'isostéaryle, palmitate d'isopropyle ou myristate d'isopropyle, des huiles minérales, des cires naturelles ou des alcools de Guerbet, des agents de conservation, des stabilisants qui empêchent une séparation de phases (désémulsification), des régulateurs de l'hydratation de la peau, par exemple des acides aminés, des antioxydants, des huiles essentielles, des agents d'écoulement, des pigments, des colorants à effet de brillance et des agents de pénétration (flux enhancer).

2. Composition cosmétique selon la revendication 1, **caractérisée en ce que** le composant (a) y est présent en une dose de 0,1 à 20 % en poids, par rapport au poids total de la composition.

3. Composition cosmétique selon la revendication 1 ou 2, **caractérisée en ce que** le composant (b) y est présent en une dose de 0,1 à 5 % en poids par rapport au poids total de la composition.

4. Composition cosmétique selon l'une quelconque des revendications 1 à 3, **caractérisée en ce que** le composant (c) y est présent en une dose de 0,1 à 5% par rapport au poids total de la composition.

5. Composition cosmétique selon la revendication 1, **caractérisée en ce que** l'agent actif approprié pour un cosmétique de la peau est choisi parmi le groupe constitué des vitamines solubles dans l'huile pouvant être appliquées par voie dermique, des huiles cosmétiques ou thérapeutiques et des agents de protection contre la lumière solubles dans l'huile du type absorbant de UVA et UVB.

6. Composition cosmétique selon l'une quelconque des revendications 1 à 5, **caractérisée en ce que** l'ester partiel d'acide gras du polyoxyéthylènesorbitane (b) est le monooléate de polyoxyéthylène-(20)-sorbitane.

7. Composition cosmétique selon l'une quelconque des revendications 1 à 6, **caractérisée en ce que** le phospholipide (c) est la lécithine purifiée, provenant de germes de soja.

8. Utilisation de la composition cosmétique selon l'une quelconque des revendications 1 à 7 dans un spray de dosage.

9. Utilisation selon la revendication 8, **caractérisée en ce que** le spray de dosage est un spray à pompe.

10. Procédé pour la préparation d'une composition cosmétique selon l'une quelconque des revendications 1 à 9, **caractérisé en ce qu'**on dissout le composant (c), le phospholipide, dans le composant (d), l'éthanol, et qu'on ajoute ensuite le composant (b), l'ester partiel d'acide gras du polyoxyéthylènesorbitane, ainsi que le composant (a), l'agent actif, et qu'on ajoute la phase huileuse ainsi obtenue, comprenant le cas échéant des composants (f) lipophiles dans la phase aqueuse, contenant le cas échéant des composants hydrophiles (f), le composant (e) et qu'on agite.

11. Composition cosmétique sous forme d'une nanodispersion, pouvant être obtenue selon le procédé selon la revendication 10.
